# EUROPEAN PATENT SPECIFICATION

(11) **EP 4 346 722 B1**
(45) Date of publication and mention of the grant of the patent: **14.05.2025**
(21) Application number: 21730901.2
(22) Date of filing: 03.06.2021
(51) Int. Cl.: A61F 13/08, A41B 11/14, A41D 13/00

(54) **COMPRESSION GARMENT**
KOMPRESSIONSKLEIDUNG
VÊTEMENT DE COMPRESSION

(43) Date of publication of application: 10.04.2024
(73) Proprietor: Essity Hygiene and Health Aktiebolag, 405 03 Göteborg (SE)
(72) Inventor: VOSS, Verena, 22761 Hamburg (DE); PLATZ, Sascha, 46446 Emmerich am Rhein (DE); BANNWARTH, Sebastian, 22761 Hamburg (DE)
(74) Representative: Nederlandsch Octrooibureau
(86) International application number: PCT/EP2021/064951
(87) International publication number: WO 2022/253440

(56) References cited:
- WO-A1-2009/114899
- WO-A1-2020/207600
- US-A- 4 027 667
- ANONYMOUS: "JOBST Measurement Guidelines", 1 November 2011 (2011-11-01), XP055893260, Retrieved from the Internet <URL:https://www.lunamedical.com/wp-content/uploads/2020/10/JobstMeasurementGuidelines.pdf> [retrieved on 20220217]
- ANONYMOUS: "FALKE Strumpfhose Family Baumwolle", 21 February 2010 (2010-02-21), XP055893332, Retrieved from the Internet <URL:https://www.amazon.de/FALKE-Babys-Strumpfhosen-Leggings-Family/dp/B005C2Z3F8/ref=sr_1_12?keywords=FALKE+Baby+Strumpfhose+offwhite&qid=1645127957&sr=8-12&th=1> [retrieved on 20220217]

## Description

### TECHNICAL FIELD

The present disclosure relates to a compression garment comprising a pants section, for example for use in the treatment of lymphatic diseases.

### BACKGROUND

Garments which are able to apply pressure to a body part of a subject are known as compression garments and have been used for a variety of therapeutic and non-therapeutic applications, such as treating lymphedema, enhancing athletic performance or for cosmetic purposes. The application of pressure to the affected body part can alleviate symptoms of lymphatic disease and prevent or slow disease progression.

A compression garment is for example disclosed in WO2009/114899 A1.

Prerequisite for a successful compression therapy is a proper fit of the garment. Garments which fit poorly on the affected body part will reduce adherence of the patient, i.e. reduce the amount of time in which the patient is wearing the garment, and will also not produce the desired compression level in all areas of the affected body part. In particular for strongly affected body parts, which can have a large diameter and an uneven surface morphology, it can be difficult to ensure a good, long-lasting fit of the garment. For such body parts, it has, thus, become routine to manufacture customized garments which are specifically adapted to the body part by manufacturing the garments based on measurements of the respective body part.

One body area, in which it is frequently difficult to ensure a proper and comfortable fit of compression pants or tights, is the area of the buttocks, hips and belly. Patients suffering from lipedema in this area often have a hip and belly area with a large circumference in comparison with a rather small waist. At present, the rear side of compressive pants can comprise two individual pieces of a knitted fabric, each covering one buttock, which are connected with a seam that is adapted to be located approximately over the intergluteal cleft of the wearer. Alternatively, the front and the back of the pants are knitted independently and connected with seams on the sides of the wearer. According to patient's feedback, prior art seams are often perceived as annoying, rubbing and sometimes uncomfortable when sitting because tension can appear on the seam. Seams are less flexible than the knitted material, especially if two seams overlap. Moreover, current products only have little adaptation to the anatomical shape of men and women, as the upper part is regularly knitted into a tube-like shape. This not only leads to a lack of comfort when the garment is worn, but can also result in an irregular compression profile along the leg.

There is, thus, a need in the art for well-fitting compression pants with an improved, i.e. accurate, compression profile along the length of the leg, buttock, hip and belly region of a wearer.

### SUMMARY

This problem is solved by the present invention as described in the appended claims. The compression garments according to the present invention comprise a pants section with a rear side that has a seamless insert covering at least part of both buttocks (butt cheeks). The width as well as the shape of the insert can be adapted to the size and shape of the wearer's buttocks, thus, providing a highly comfortable and therapeutically accurate garment.

In a first aspect, the invention relates to a compression garment comprising a pants section with a rear side, wherein the rear side comprises an insert, a first side section and a second side section, the first side section being connected to a first side of the insert, the second side section being connected to a second side of the insert, wherein the insert is adapted to extend over at least part of both buttocks of a wearer, wherein the insert consists of a seamless knitted fabric; and wherein the insert is located between the first side section and the second side section and a waistband section; or the insert is located between the first side section and the second side section and forms part of the upper end of the garment.

The compression garment of the invention is suitable for use in the treatment or prevention of various diseases or conditions which require treatment or prevention with compression therapy. These diseases and conditions include diseases and conditions treated with garments of Compression Class 1, Compression Class 2, Compression Class 3 or Compression Class 4. Diseases and conditions treated with garments having Compression Class 1 (CCL 1) can be e.g. selected from the group consisting of tired, heavy, aching legs; minor varices without edema; mild swelling of the feet, ankles and legs; onset of pregnancy-related varices; primary or secondary lymphedema in International Society of Lymphology (ISL) stages I-III; and lipedema. Diseases and conditions treated with garments having Compression Class 2 (CCL 2) can be e.g. selected from the group consisting of tired, heavy, aching legs; moderate to severe varices with and without edema; moderate to severe pregnancy-related varices with and without edema;·moderate edema; chronic venous insufficiency (CVI) C3-C5 according to CEAP; venous edema, skin alterations; healed ulcus cruris; venous ulcers; superficial thrombophlebitis / SVT; diseases and conditions resulting from vein surgery; diseases or conditions resulting from sclerotherapy; post-traumatic edema; post-operative edema; primary or secondary lymphedema in ISL stage II; phlebolymphedema; lipedema. Diseases and conditions treated with garments having Compression Class 3 (CCL 3) can e.g. be selected from the group consisting of severe varices with or without edema; severe edema; chronic venous insufficiency (CVI) C3-C6 according to CEAP; venous edema; skin alterations; healed ulcus cruris; ulcus cruris; venous ulcers; post-thrombotic syndrome (PTS); superficial thrombophlebitis / SVT; conditions or diseases after vein surgery; conditions or diseases after sclerotherapy; primary or secondary lymphedema in ISL stage II; risk of rapid edema rebound; and lipedema. The lymphedema can be e.g. lymphedema with or without shape distortions.

Described herein but not part of the invention are methods for the treatment or prevention of any of the aforementioned diseases by wearing the compression garment described herein as well as the compression garment for use in a method of treatment or prevention of any of the aforementioned diseases by wearing the compression garment.

Depending on the location and extent of the disease, patients require compression therapy in different areas of the lower body. The compression garment can, e.g., be pants or tights, wherein pants can be of different lengths and the tights can e.g. be open-toe or closed-toe tights. The compression garment can also be a hybrid of pants and tights, i.e. a pant-like structure having one closed-toe leg such as a tight and one pants leg. The terms "tights" and "pantyhose" are used synonymously herein. Additionally, the terms "pants" and "pair of pants" are used synonymously herein.

The upper (cranial / proximal) end of the pants or tights can be adapted to end in the hip or waist region of the wearer or it can reach up to the rib region. In the area of the legs, i.e. the distal part of the pants, the pants can be adapted to end around the thighs, e.g. the proximal or distal part of the thighs, proximally or distally from the knee, around the lower leg, e.g. in the proximal or distal part of the lower leg, proximally or distally from the ankle or around the feet. Some patients require the compression treatment only or predominantly in one leg and not in the other. A garment with leg sections of different lengths is, therefore, also possible. Any combination of the aforementioned embodiments of pants or tights is within the scope of this invention. The garment may, e.g. have one leg in a closed-toe tights configuration and a second leg that is adapted to end proximally of the knee of the wearer.

The garment is suitable to exert a therapeutic, pre-determined pressure on the body part or the body parts on which it is worn, when worn correctly. For this purpose, the garment is preferably a customized compression garment, i.e. manufactured taking the circumferences and lengths of the legs and lower torso of the wearer into account. Nevertheless, the garment can also be a premade, i.e. off-the-shelf, garment. The wearer, i.e. the patient in need of treatment, would then choose the garment that has the best fit. The "wearer" mentioned herein is usually a patient, e.g. a patient in need of compression therapy in the area of the legs, buttocks, hips, belly and/or waist.

The garment can, e.g. be a compression garment having compression class (CCL) 1, 2, 3 or 4. The pressure value at the ankle in these compression classes is 18-21 mmHg (class 1), 23-32 mmHg (class 2), 34-46 mmHg (class 3) and at least 49 mmHg (class 4). Classification of the garment into these compression classes can be done according to RAL-GZ 387/1. Preferably, the garment is a compression class 1, 2, 3, or 4 garment, most preferably a compression class 1, 2 or 3 garment.

The garment comprises a pants section with a rear and a front side. Each side is adapted to fit the respective side of the wearer's body in the lower torso area, in particular the pelvis area, and upper area of the legs. The rear side of the pants section is, hence, adapted to fit / cover a section of the rear side of the wearer's body, specifically, the buttocks and the rear of a proximal part of the legs of the wearer. The rear side of the garment comprises an upper section that is adapted to cover the buttocks and the intergluteal cleft of the wearer and a lower part adapted to cover at least the upper part of the legs of the wearer. The garment can, e.g. be pants or tights as mentioned above.

The rear side comprises an insert, a first side section and a second side section. Each of these parts - the insert, the first side section and the second side section are different pieces of fabric or parts of different pieces of fabric. In other words, the insert, the first side section and the second side section are not part of the same seamless piece of fabric. To form the garment, they have to be connected first, e.g. by seams as outlined elsewhere herein. A "seam" as used herein and in the usual sense of the word is a line where two or more pieces of knitted fabric are held together by (sewn) stitches.

In one embodiment, the insert as described herein is a first piece of knitted fabric, the first side section a part of a second piece of knitted fabric, and the second side section a part of a third piece of knitted fabric. The insert could only be present in the rear part of the garment and not in the front. The insert could end approximately in the middle of the crotch area.

Alternatively, the insert could extend to the front part of the garment, in particular in garments adapted to fit a male person. In such an embodiment, the insert, the first side section and the second side section are parts of a first piece, a second piece and a third piece of knitted fabric, respectively. The insert can be present only in the rear part of the garment or also be present in (extend to) the front side of the garment, e.g. extend from the rear part through the rear and front part of the crotch area into the front part of the garment.

In both described embodiments, the first, second and third pieces of knitted fabric are distinct, individual pieces (in other words: seamless) and have to be connected to each other and, potentially, to further pieces of knitted fabric to form the compression garment. The pieces from which the compression garment is formed can be knitted into the desired shape and size or cut from a larger piece of knitted fabric.

The insert is adapted to extend over (cover) at least part of both buttocks of a wearer, e.g. 10% or more of the surface area of each buttock, 15% or more, 20% or more, 25% or more, 30% or more, 35% or more, 40% or more. In other words, the insert can be adapted to cover from 10% to 60%, from 15% to 60%, from 20% to 60%, from 25% to 60%, from 30% to 60%, from 35% to 60%, or from 40% to 60% of the surface area of each buttock of the wearer.

The insert can make up for 30 % or more of the area of the compression garment in the rear side of the pants section, e.g. 35% or more, 40% or more, 45% or more, 50% or more. In other words, the insert can make up for from 30% to 75%, from 35% to 75%, from 40% to 75%, from 45% to 75%, from 50% to 75% of the area of the compression garment in the rear side of the pants section. The insert making up for e.g. 30% or more of a certain area of the compression garment means that the surface area of the insert covers 30% or more of the surface area of the respective area of the compression garment.

The insert is, further, adapted to extend over at least part of the intergluteal cleft of the wearer. This means that the insert is substantially in the middle of the rear side of the pants section of the garment. In transverse direction (from proper left to proper right of the garment), the insert is located between the first side section and the second side section. In longitudinal direction, the insert is located between leg sections of the garment and, potentially, a waistband section. The "waistband section" is the rear part of a waistband. The waistband usually spans around the entire waist. When no waistband is added to the garment, the insert can form part of the upper end of the garment.

To distinguish the left and the right part of the garment, the terms "proper left" and "proper right" are used herein. The "proper left" is the side of the garment that would be regarded by the wearer as the left side when the garment is worn correctly (i.e. with the insert covering at least part of both buttocks). The "proper right" is the side of the garment that would be regarded by the wearer as the right side when the garment is worn correctly.

The garment can comprise two leg sections - a first leg section adapted to cover at least part of the proper left leg of the wearer and a second leg section adapted to cover at least part of the proper right leg of the wearer. The first and the second leg sections have at least partially the shape of a tube and are located distally from the pants section. Each leg section encircles at least part of a leg.

Because the - seamless - insert is covering at least part of the intergluteal cleft of the wearer, the garment comprises no seam in the area covered by the insert. The insert can be adapted to cover the entirety of the intergluteal cleft of the wearer. The insert consists of a seamless knitted fabric, preferably a flat-knitted fabric. The term "seamless" means that the fabric does not comprise any seams in the respective area. Usually, this will be achieved by knitting the respective seamless piece in the desired shape and size on a knitting machine or by cutting the respective piece of knitted fabric from a larger piece of knitted fabric.

The different pieces of the garment (e.g. the insert, the first side section and the second side section) can be knitted fabric, such as a flat-knitted or a circular knitted fabric. They are, in other words, comprising or consisting of seamless knitted fabric. Preferably, like the insert, the first side section and the second side section each consist of seamless knitted fabric. The knitted fabric of the insert as well as the other pieces from which the garment is manufactured can be a double-layer knitted fabric, which can e.g. be manufactured on a knitting machine having at least two needle beds. The two layers of the knitted fabric can have different, complementing properties that can be created by using particular yarns and/or knitting techniques. It has, for example, been proven particularly advantageous to use a knitted fabric comprising a hydrophobic and a hydrophilic yarn, such as the knitted fabric described in WO 2020/207600 A1.

The forming of particular stitches with these yarns on the needle beds of the knitting machine can create a knitted fabric that is able to transport liquid from the inside of the garment to the outside.

The insert has a shape that allows it to be connected to the first side section and to the second side section. The side sections are connected to the insert on opposite sides of the insert (opposite in transversal direction of the rear side). In other words, the insert is located between the two side sections. The side sections are preferably not connected to each other or otherwise adjacent to each other in the rear side of the pants section. The first side section is connected to a first side of the insert, and the second side section is connected to a second side of the insert. The first side of the insert can be substantially opposite the second side of the insert. This means that the first and second side sections of the garment are connected to substantially opposite sides of the insert. The phrase "sides of the insert" refers to the edges of the insert. It does not refer to the extended surface areas which are parallel to the wearer's skin, when the garment is worn.

Preferably, the first and second side sections are located on different sides of the median plane of the garment. In line with the standard use of the term, the "median plane" of the garment divides the garment - and its rear side - into a left and right part.

The first side section can, for example, be on the "proper left" side of the rear part (i.e. the side that would be regarded by the wearer as being on the left side of the garment when worn) and the second side section can, for example be on the "proper right" side of the rear part (i.e. the side that would be regarded by the wearer as being on the right side of the garment when worn). Preferably, the first and second side sections are not adjacent to each other, but fully separated from each other by the insert as mentioned above.

In other words, the first side section can be adapted to cover a part of a first buttock of the wearer (e.g. the buttock on the "proper left" of the wearer) and the second side section can be adapted to cover a part of a second buttock the wearer (e.g. the buttock on the "proper right" of the wearer).

The first and second side sections will usually be connected to the insert by means of a seam. Thus, in some embodiments, the first side section is connected to the first side of the insert by means of a first seam and the second side section is connected to the second side of the insert by means of a second seam. The skilled person will be able to find a seam that is suitable to join these pieces of the compression garment. The seams which are used to connect the different parts of the compression garment are preferably relatively flat, in particular on the surface of the garment that is worn on the skin of the wearer (the "inner surface"). Particularly preferred seams are flat on the inner as well as the outer surface of the garment. Thereby, the wearing comfort of the garment as well as its visual appearance is enhanced.

The knitted fabric in the respective seam can be overlapping, but is preferably not folded. In other words, the knitted fabric from the first or second side portion can overlap with the knitted fabric of the insert in the area of the seam. The knitted fabric will then be connected with one or more threads. The resulting seams can be termed overlapping seams. It is not necessary to fold the fabric in this area as this would thicken the seam to an undesirable extent. Alternatively, the knitted fabric from the first or second side portion can merely contact each other without overlap. In other words, the seams can alternatively be abutted seams.

The one or more threads used for making the seam can be standard yarns used in the art. In one embodiment, the seam can have a thickness of up to 230% of the thickness of the side portions, preferably of up to 220% or less, such as 215%. In other words, the seam can have a thickness of from 200% to 230% of the thickness of the side portions, preferably of from 210% to 230%, such as of from 220% to 230%. It will be understood that the thickness of the fabric in a middle region of each side portion is 100%. Due to the non-folded overlap of the fabric from each side portion in seams with overlapping knitted fabric, the thickness in the seam area will be doubled, i.e. be 200% of the thickness in a middle region of the side portion. The one or more threads used will add to this thickness in the range of 10% to about 30%. Accordingly, in abutted seams, the seam can have a thickness of up to 130% of the thickness of the side portions, preferably of up to 120% or less, such as 115%. In other words, the abutted seam can have a thickness of from 100% to 130% of the thickness of the side portions, preferably of from 110% to 130%, such as of from 120% to 130%.

The seam can be generated with an overedge chain stitching technique. This has the advantage that the resulting seam will be flexible as well as durable and comfortable to wear. The overedge chain stitching technique is preferably selected from the group consisting of overedge chain stitching type 607 and 605, preferably 607. These stitching types are known in the art. They belong to the class 600 of cover-seam chain stitches.

The seams which connect the insert and the side sections can be arranged to be substantially in the middle of the respective buttock. More importantly, they are designed to be continuous with a seam in the rear part of the respective leg section. The seam, in other words, runs from the proximal part of the rear side of the pants section in distal direction over the part of the garment that is adapted to cover the respective buttock into and along the respective leg section in distal direction. The respective seam can be a substantially straight seam, in particular a seam that looks substantially straight when viewing the garment from behind the wearer. This does, of course, not exclude that the seam adapts in a form-fitting manner to the bumps in the area of the buttocks and the rear part of the legs of the wearer. When viewed from the side, the seam may, thus, not look straight.

The leg sections of the garment can each comprise a seam running in longitudinal direction substantially in the middle of the rear of the leg section. Preferably the leg sections (including front and rear of the respective leg section) do not comprise further seams running in longitudinal direction, more preferably they do not comprise any further seams - with the exception of potential closing seams at the distal end of the leg sections of the garment. The garment does, in these cases not comprise any seams on the front side of the garment in the area of the legs of the wearer, with the exception of potential closing seams at the distal end of the leg sections of the garment.

The leg sections of the garment are, in other words, each preferably manufactured from a single piece of knitted fabric that is closed in the back of the respective leg with a seam, e.g. in the middle of the leg. This design is particularly comfortable because it reduces the number of seams considerably. Additionally, the respective pieces of knitted fabric can be manufactured in a customized manner on e.g. flat-knitting machines and, therefore, specifically adapted to the shape and size of the wearer. The leg sections can, for example, be knitted substantially as described in WO 2019/224204 A1, which is incorporated herein in its entirety.

The insert comprises or consists of a proximal and a distal part. The distal part is connected to the leg sections of the garment and can extend into the crotch area of the garment, i.e. the area which is adapted to cover the crotch of the wearer.

It has been found in the context of this invention that a particularly good fit of the garment is ensured when connection endpoints E1 and E2 are each located at a particular height within the garment. The connection endpoints E1 and E2 are the points on the outer circumference of the insert which mark the end of the connection to the first side section and the second side section, respectively. Proximally from the connection endpoints E1 and E2, respectively, the first and second seams are connecting the first side section and second side section, respectively, to the insert. Distally from the connection endpoints E1 and E2, the continuous first and second seams are connecting two sides of the respective leg section with each other.

The part of the insert located proximally from connection endpoints E1 and E2 can be regarded as the proximal part of the insert; the part of the insert located distally of the connection endpoints E1 and E2 can be regarded as the distal part of the insert.

Distally from connection endpoints E1 and E2, the insert is shaped in a manner so that it can extend into the crotch area. The distal part of the insert comprises a distal rear section and a crotch part. The crotch part of the insert is termed "crotch part of the insert" herein because it is adapted to extend into the crotch area. In the crotch part of the insert, the sides of the insert can also be connected to the respective leg sections of the garment, in particular to the part of the leg sections which is adapted to be worn on the inner side of the respective leg.

The insert can have a maximal width (W) that is 30% or more of the maximal width (W') of the rear side, such as 35% or more, 40% or more, or 45% or more. In other words, the maximal width (W) of the insert can be from 30% to 60% of the maximal width (W') of the rear side, from 35% to 60%, from 40% to 60%, from 45% to 60%, or from 45% to 55%. The maximal width is the width measured in transverse direction, i.e. substantially parallel to the upper end of the garment. As the stitch courses in the knitted fabric will usually also run in transverse direction, the maximal width can also be the longest stitch course in the insert. The maximal width can be between the connection endpoints E1 and E2. Depending on the wearer's shape (e.g. the difference in circumference in hip and waist area), the maximal width can, however, also be in another part of the insert. In this embodiment, the stitch course between connection endpoints E1 and E2 is shorter than the stitch courses distally and proximally therefrom.

The stitch course within the insert that is adapted to be located in the middle of the crotch area of the wearer (in anterior to posterior direction) is termed "middle course". In the crotch area, in particular at the middle course, the insert is narrower (i.e. courses comprise less stitches) than in the distal rear section and the proximal rear section, in particular narrower than the distance between the connection endpoints E1 and E2.

The insert comprises at least three sections which are knitted in a single piece. In particular, the insert comprises a crotch part, a distal rear section and a proximal rear section. Crotch part and distal rear section form the distal part of the insert mentioned above. The proximal rear section can form the proximal part of the insert mentioned above. The virtual line between connection endpoints E1 and E2 separates the distal from the proximal rear section. As outlined above, the proximal rear section up until and including connection endpoints E1 and E2 is connected to the first side section and the second side section.

The first side section can be a part of a first seamless hip-to-leg portion, the second side section can be a part of a second seamless hip-to-leg portion, and the first and second hip-to-leg portions are each adapted to extend from the hip region of a wearer to at least the thigh region of the wearer. These hip-to-leg portions can each be a single piece of knitted fabric. The hip-to-leg portions can, in other words, also be termed hip-to-leg parts. They can, in preferred embodiments, be large enough to extend from the rear side of the garment to the front. The hip-to-leg portions can comprise the above-mentioned side sections as well as the leg sections. In other words, the first leg section can be a part of a first seamless hip-to-leg portion and the second leg section can be a part of a second seamless hip-to-leg portion. In these embodiments, the leg sections of the hip-to-leg portions are each adapted to wrap around the leg of a wearer and formed into a tube-like structure with a seam in the middle of the rear part of the leg section.

The first seam and the second seam - which are described elsewhere herein and which connect the side sections to the insert - can each extend from the pants section into a first leg section and a second leg section, respectively. The leg sections are each adapted to cover at least part of a leg of a wearer. In the first leg section, the first seam can connect a first side of the first hip-to-leg portion with a second side of the first hip-to-leg portion. This part of knitted fabric in the garment is, in other words, forming a first tube-like structure. The first seam runs continuously from the proximal (cranial) part of the garment to the distal end of the garment. The same applies to the second seam, which can connect a first side of the second hip-to-leg portion with a second side of the second hip-to-leg portion.

The proximal and distal rear sections of the insert are preferably connected to different, non-adjacent sides of the piece of knitted fabric comprised in the hip-to-leg portions. The non-adjacent sides connect in the connection endpoints E1 and E2.

The particular placement and shape of the insert, which is described elsewhere herein in more detail, and the use of hip-to-leg-portions reaching from the waistline to the distal end of the garment prevents constriction in the transition zone between the leg sections and the pants section. Prior art garments are often not ideally adapted to the wearers body shape in this transition zone or the different garment parts do not fit together perfectly. Moreover, in the manufacture of a garment according to the present invention, the dimensions of the leg sections can be determined in the same calculation step as those of the pants section. In other words, the method for manufacture of a compression garment according to the invention (described in more detail elsewhere herein) can comprise a step of calculating the dimensions of the hip-to-leg portions. The calculation could take into account at least one length and/or at least one circumference of the wearer's leg in the area of the leg section of the pants section as well as at least one length and/or circumference of the wearer's leg in the area of the respective leg section of the garment. Thereby, it can be ensured that the proximal end of each leg section is attached harmoniously to the pants section. In contrast, in present state of the art calculations, the pants sections are often calculated separately from the leg sections and it cannot always be guaranteed that the upper end of the leg sections (standard measurement value cG) fits the respective counterpart of the pants section properly.

The length of the crotch part of the insert and the distal rear section of the insert is dependent on the circumference of the garment in the thigh region. In other words, the lengths of the crotch part of the insert and the distal rear section of the insert are adapted to have the same length as the side of the knitted fabric of the hip-to-leg portion that is connected to these parts of the insert. The lengths can be given e.g. in cm or number of stitch courses.

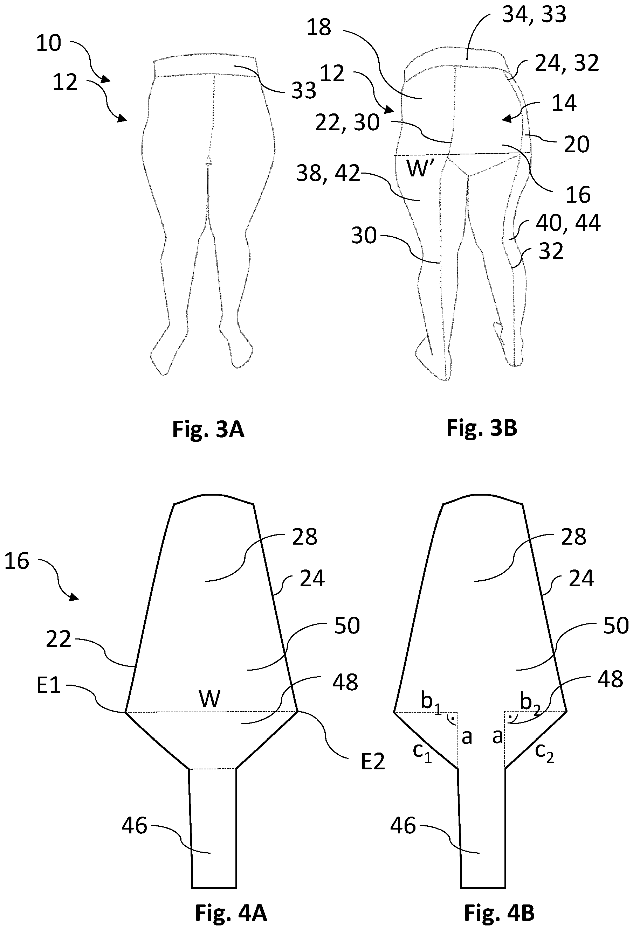

The optimal distance between the connection endpoints E1 and E2 in the insert, i.e. the width of the piece of seamless knitted fabric at this position can be calculated using the Theorem of Pythagoras (c₁² - a² = b₁²) and (c₂²- a² = b₂²), with c₁ and c₂ being the lengths of the (lateral) sides of the distal rear section of the insert as measured between the respective connection endpoint E1 or E2 and the respective start of the crotch part of the insert; and a being the inner length of the distal rear part, measured from crotch part to proximal rear section in a direction that is parallel to a longitudinal axis of the insert (i.e. in stitch wale direction, i.e. orthogonally to the stitch courses); and b₁ + b₂ + crotch part width = distance between E1 and E2 (compare Fig. 4B). The inner length of the distal rear part a can be a = (IH (hip height) - IK (crotch height)) * M (geometic body shape modifier), wherein M is between 0.5 and 0.95.

The insert can extend beyond the middle of the crotch area to the front part of the garment. Depending on the needs of the wearer, the part of the insert which is located in the front of the garment can be shorter or longer. For example, the garment can be adapted to be a garment for male wearer or a garment for a female wearer. In particular for female wearers it is not necessary to add additional pieces of knitted fabric to the front of the garment, but the insert can nevertheless have a short front section. Should the garment, however, be adapted to be worn by a male wearer, then it has been found to be advantageous if the compression garment comprises an additional piece of knitted fabric as a second insert for the front. This additional piece of knitted fabric will be termed "fly part" herein.

The fly part is connected to the hip-to-leg portions described herein in the front part of the compression garment. In particular, the fly part is between the first and the second hip-to-leg portion. The connections may again be made with suitable seams. In addition, in one embodiment, the fly part can be connected to the insert in the crotch area with a seam. In an alternative embodiment, the insert and the fly part can be arranged to overlap in the front part of the compression garment, thus, creating a fly opening. In preferred embodiments, the fly opening is tilted, i.e. neither parallel to a transverse nor to a longitudinal axis of the garment. On its proximal end, the fly part can be connected to a waistband.

The compression garment can comprise a waistband. Accordingly, the rear side of the compression garment, may comprise a waistband section. The "waistband section" is the part of the waistband that adapted to be worn on the rear side of the wearer. The waistband and its waistband section can form the proximal (cranial) end of the garment. The waistband can run around the entire front and rear side of the garment. Thereby, a particularly good fit of the garment can be ensured. It is also possible to equip the waistband section with sticking properties. It can, for example be knitted with yarns having high friction.

To ensure a particularly good fit of the garment, the number of pieces of knitted fabric which are connected to form the garment and, thus, the number of connecting seams, is preferably kept to a minimum. The rear side of the garments can, therefore, consist of the insert, the first side section, the second side section, the waistband section and seams connecting the aforementioned sections and insert. A male compression garment may consist of the insert, the first hip-to-leg-portion, the second hip-to-leg-portion, the waistband, the fly part and seams connecting the aforementioned sections and insert. A female compression garment may consist of the insert, the first hip-to-leg-portion, the second hip-to-leg-portion, the waistband and seams connecting the aforementioned section, part and insert. Each of the aforementioned parts can be a single piece of seamless knitted fabric.

In a second aspect, the present invention relates to a method for determining measurement information for manufacturing a compression garment, comprising a) selecting a wearer in need of compression therapy with compression pants and/or compression tights; b) acquiring measurement information from the wearer, wherein the measurement information comprises: i. optionally, circumference measurements obtained in the torso, hip, and/or leg area; ii. optionally, length measurements obtained in the torso, hip, and/or leg area; and iii. a measurement of the total rise length (IL), and a simultaneous measurement of the front rise length (IL1); and c) optionally, manufacturing a compression garment based on the measurement information acquired in step (b) or selecting a pre-fabricated compression garment based on the measurement information acquired in step (b).

The "total rise length" is the distance between a starting point in the middle of the anterior waistline and an endpoint in the middle of the posterior waistline, wherein the measurement is taken through the crotch area. The "front rise length" is the distance between the starting point in the middle of the anterior waistline and the middle of the crotch area.

The measurements, in particular the measurement in step (b)(iii) can be made with a measuring tape comprising a slider. The slider is movably attached to the measuring tape. This means that the slider can be moved along the measuring tape to different positions, i.e. different positions at the scale of the measuring tape. While the slider is movable along the scale, it will not slide on its own. Instead it will maintain its position on the scale to which it has been moved by the medical practitioner performing the measurement (fitter) or by the wearer himself/herself. During the measurement in step (b)(iii), the medical practitioner performing the measurement can place the slider in the middle of the crotch area of the wearer. While the slider is moved into the middle of the crotch area, one end of the scale on the measuring tape is held to the starting point in the middle of the anterior waistline of the wearer. It is particularly advantageous that the slider can be moved and placed in the middle of the crotch area by the wearer herself/himself because there is no need for the medical practitioner to touch the intimate crotch area of the wearer.

After moving the slider to the middle of the crotch area, the medical practitioner can guide the measuring tape to the endpoint in the middle of the posterior waistline while maintaining the end of the scale on the measuring tape on the starting point in the middle of the anterior waistline and/or maintaining the slider in the middle of the crotch area.

Accordingly, a "simultaneous" measurement of the total rise length (IL) and the front rise length (IL1) means that at least part of the measuring instrument (e.g. the measuring tape) is kept constant relative to the wearer's body during both measurements. In preferred embodiments, the simultaneous measurements in step (b)(iii) use a measuring tape comprising a slider and the slider is placed in the middle of the crotch area.

The acquisition of measurement information in step (b)(ii) can comprise measuring the length of the crotch height (IK). As is well known in the art, the crotch height is the distance from the soles of the wearer (or the floor on which the wearer stands) to the middle of the crotch area.

The acquisition of measurement information in step (b)(ii) can comprise measuring a hip height (IH) in the hip area, wherein the hip height (IH) is at least 3 cm higher than the crotch height (IK) and, preferably, taken at the hip height where the hip has the largest circumference. The hip height is the distance from the soles of the wearer (or the floor on which the wearer stands) to the aforementioned point of the hip with the largest circumference, wherein this point is at least 3 cm higher than the crotch height.

In a third aspect, the invention relates to a method for manufacturing a compression garment, preferably a compression garment as described elsewhere herein, comprising a) obtaining measurement information from a wearer, preferably as described elsewhere herein, or obtaining measurement information for a pre-designed compression garment; b) manufacturing the compression garment based on the measurement information, by connecting three or more pieces of seamless knitted fabric and thereby forming the compression garment, wherein the three or more pieces of seamless knitted fabric include at least a piece of seamless knitted fabric forming an insert; a piece of seamless knitted fabric forming a first hip-to-leg portion; a piece of seamless knitted fabric forming a second hip-to-leg portion; and optionally, a piece of seamless knitted fabric forming a waistband section; and wherein compression garment fabricated in step (b) comprises a pants section with a rear side, wherein the rear side comprises the insert, a first side section and a second side section, the first side section being connected to a first side of the insert, the second side section being connected to a second side of the insert, and wherein the insert is adapted to extend over at least part of both buttocks of a wearer. In other words, the compression garment has the properties defined herein for the compression garment of the present invention.

As explained in more detail elsewhere herein, the knitted fabric can be a double layer knitted fabric. For example, the knitted fabric can be manufactured in a step (a1) on a knitting machine having at least two needle beds (V, H), wherein the knitted fabric comprises a first layer made of a hydrophilic yarn on the first needle bed (V), a second layer made of a hydrophobic yarn on the second needle bed (H) and the second layer comprises spaced apart split stitches made of the hydrophilic yarn. This configuration ensures a good transport of the liquid to the outside of the garment, away from the skin of the wearer.

### BRIEF DESCRIPTION OF FIGURES

Exemplary embodiments of the invention are shown schematically in the drawings.
- **Fig. 1**: schematically shows (A) an oblique frontal view and (B) an oblique rear view of a compression garment of the invention adapted to be worn by a female wearer;
- **Fig. 2**: schematically shows (A, B) oblique frontal views and (C) an oblique rear view of a compression garment of the invention adapted to be worn by a male wearer, wherein (A) shows an embodiment without a fly opening and (B) shows an embodiment with a fly opening;
- **Fig. 3**: schematically shows (A) an oblique frontal view and (B) an oblique rear view of a compression garment of the invention adapted to be worn by a female wearer, wherein the compression garment has a bigger size than the one shown in Fig. 1;
- **Fig. 4**: schematically shows (A, B) the shape of a piece of knitted fabric that forms the insert, i.e. shows the shape of one embodiment of the insert;
- **Fig. 5**: schematically shows (A to I) oblique frontal views of different embodiments of the compression garment of the invention having different leg lengths and closed-toe (A) and open toe configuration (B to I), wherein each embodiment is adapted to be worn by a female wearer;
- **Fig. 6**: schematically shows (A to I) oblique rear views of different embodiments of the compression garment of the invention having different leg lengths and closed-toe (A) and open toe configuration (B to I), wherein each embodiment is adapted to be worn by a male wearer; and
- **Fig. 7**: schematically shows the new total rise length (IL) measurement for compression garments according to the invention.

These and other aspects of the invention will be apparent from and elucidated with reference to the embodiments described hereinafter.

### DESCRIPTION OF EMBODIMENTS

Additional advantages, characteristics, and features of the present invention will become clear from the following detailed description of exemplary embodiments with reference to the attached drawings. However, the invention is not restricted to these exemplary embodiments.

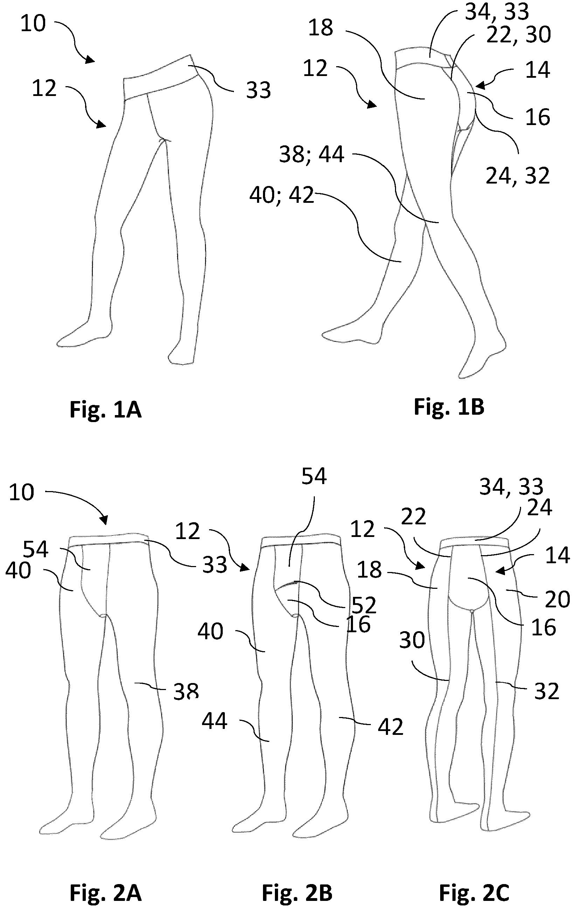

Fig. 1 schematically shows (A) an oblique frontal view and (B) an oblique rear view of a compression garment 10 of the invention adapted to be worn by a female wearer. The compression garment 10 consists of a waistband 33 (comprising a rear waistband section 34), a first hip-to-leg portion 38, a second hip-to-leg portion 40, an insert 16 located at the rear side of the garment 10 and seams, e.g. 30, 32, connecting the aforementioned parts. Each of the waistband 33, the first hip-to-leg portion 38, the second hip-to-leg portion 40, and the insert 16 consist of a single seamless piece of knitted fabric. The knitted fabric can, for example, be a flat-knitted or circular-knitted piece of fabric. In both cases, it can be a double-layer knitted fabric.

The compression garment 10 has the form of tights (pantyhose) with a closed-toe configuration on both foot parts. The tights comprise a pants section 12 having a rear side 14 and a front side. The rear side 14 of the pants section comprises the insert 16, a first side section 18 and a second side section 20. It can be seen that the first and the second side sections 18, 20 are part of the first and second hip-to-leg portion 38, 40, i.e. a part of the seamless piece of knitted fabric forming the respective portion.

Further to the first and second side sections 18, 20, respectively, the first and second hip-to-leg portions 38, 40, each comprise a leg section - the first and second leg section 42, 44, respectively - and a front part. These leg sections 42, 44 each extend along and around one leg of the wearer. The leg sections 42, 44 are, in other words, covering the middle and lower legs of the wearer - those sections which are not covered by the pants section 12 of the compression garment 10.

Additionally, the first and second hip-to-leg portions 38, 40, each comprise a front part which together with the front part of the waistband 33 form the front part of the pants section 12 of the tights.

It can be seen that the insert 16 is located in the middle of the rear side 14 of the pants section 12 between the first side section 18 and the second side section 20. Thereby, the insert 16 covers the intergluteal cleft as well as parts of both buttocks of the wearer. The insert is broad enough to make up for 30 % or more of the area of the compression garment 10 in the rear side 14 of the pants section 12.

The insert 16 fits snugly and comfortably on the portion of the rear of the wearer that it is supposed to cover. To achieve this purpose, the shape and dimension of the insert 16 as well as the shape and dimension of the remainder of the garment are adapted precisely to the wearer's measurements that have been taken in advance of manufacturing the customized garment. It will be understood that it is also possible to manufacture pre-fabricated garments of the same type.

A first side 22 (edge) of the insert 16 is connected to the first side section 18 with a first seam 30; a second side 24 of the insert 16 is connected to the second side section 20 with a second seam 32. Both seams 30, 32 extend further along the leg in distal direction beyond the direct connection to the insert 16. Specifically, in connection endpoints E1 and E2 (see Fig. 4), the seams 30, 32 start to connect two sides of the first hip-to-leg portion 38 and the second hip-to-leg portion 40, respectively (instead of connecting the respective side section 18, 20 located within the respective hip-to-leg portion 38, 40 to the insert 16).

Fig. 2 schematically shows (A, B) oblique frontal views and (C) an oblique rear view of a compression garment 10 of the invention adapted to be worn by a male wearer, wherein (A) shows an embodiment without a fly opening and (B) shows an embodiment with a fly opening 52. The compression garment 10 shown in Fig. 2 comprises essentially the same components as the one described above for Fig. 1 with the following slight modifications.

To accommodate the male physique, the compression garment 10 intended for a male wearer comprises a fly part 54 in the front side of the garment 10. In the embodiments shown, the fly part 54 consists of a seamless piece of knitted fabric.

The embodiment shown in Fig. 2A comprises a fly part 54 extending in longitudinal direction from the waistband 33 to the middle of the crotch area. This embodiment does not contain a fly opening. In transverse direction, the fly part is located between and connected to the first and the second hip-to-leg portions 38, 40 of the compression garment 10. In the middle of the crotch area, the fly part 54 is connected with a seam to the insert 16 (not shown). In an alternative to this embodiment, the fly part 54 can be part of the same piece of seamless knitted fabric as the insert 16.

The embodiment shown in Fig. 2B comprises a fly opening 52 in approximately the middle of the front side of the pants section. The fly opening 52 is tilted with respect to longitudinal and transverse axes of the garment. This ensures a comfortable accessibility of the fly opening for the wearer. At the same time, the fly opening stays closed when the garment is worn, in contrast to a vertical fly opening as used in standard non-compression garments. The fly opening 52 is formed by overlapping a fly part 54 with a part of the insert 16. In this embodiment, the insert 16 extends through the crotch area into approximally the middle of the front side of the pants section 12 of the garment 10. The fly part 54 extends from the waistband 33 in distal direction approximally half-way to the crotch area. Variation of the length of the fly part 54 and the insert 16 is possible. In an alternative to this embodiment, an additional fly part 54' (not shown) can replace the extended insert 16 and be connected to the insert in the middle of the crotch area and form a fly opening 52 by overlapping with the first fly part 54 (not shown).

It can be seen that the waistband 33 in Fig. 2 is narrower than the waistband 33 in Fig. 1. Depending on the wearer's physique and/or wishes, the width of the waistband 33 (i.e. the length of the waistband 33 in longitudinal direction of the garment 10) can be varied to achieve maximal comfort. For example, it has been found that women often prefer a broader waistband 33 than men.

Fig. 3 schematically shows (A) an oblique frontal view and (B) an oblique rear view of a compression garment 10 of the invention adapted to be worn by a female wearer, wherein the compression garment 10 has a bigger size than the one shown in Fig. 1. In general, the garment 10 is built as the garment 10 shown in Fig. 1 with the following variations.

It can be seen that the insert 16 in Fig. 3B is broader in transverse direction than the one in Fig. 1B and adapted to the shape of the legs, hips, buttocks, belly of a bigger wearer. The garment 10 is a customized garment specifically designed for a particular wearer in need of compression therapy.

The maximal width W of the rear side 14 of the pants section 12 of the garment 10 is longer than the maximal width W of the garment in Fig. 1. Accordingly, also the shape and dimensions of the insert 16 have been adapted to facilitate a good fit of the customized compression garment.

Fig. 4 schematically shows the shape of a piece of knitted fabric that forms the insert 16, i.e. shows the shape of one embodiment of the insert 16, when the knitted fabric 28 forming said insert is set out flat on a flat surface. Both, Fig. 4A and Fig. 4B show the same insert, but different features of the insert are labeled in A and B. The dotted lines in Fig. 4A and Fig. 4B are not part of the actual insert. Instead, the dotted lines are describing (A) the boundaries of proximal rear section 50, distal rear section 48 and crotch part 46 and (B) the lengths a, b₁ and b₂.

The insert 16 consists of a double-layered, seamless knitted fabric 28. It comprises a proximal rear section 50 (proximal part) that is located in the rear of the compression garment 10 between the first side section 18 and the second side section 20 and connected to these two side sections 18, 20 with its first side 22 and its second side 24, respectively. The distal end of the proximal rear section 50 is formed by an imaginary line connecting the connection end points E1 and E2. The maximal width W of the insert 16 is calculated as described elsewhere herein.

The insert 16 additionally comprises a distal part comprising a distal rear section 48 and a crotch part 46. It can be seen that the crotch part 46 is considerably narrower than the average width of the distal rear section 48 and the average width of the proximal rear section 50. Thereby, the crotch part 46 is adapted to be worn between the legs in the crotch area. The distal rear section 48 connects the crotch part 46 and the proximal rear section 50. The distal rear section 48 and the proximal rear section 50 in the embodiment shown here and other embodiments have a trapezoid form, in particular the form of an equilateral trapezoid.

It can be seen in Fig. 4B that the lengths c₁, a and b₁ as well as c₂, a and b₂ each form a right-angled triangle. Knowledge of two of the values (c₁ or c₂, and a) can, hence, be used to calculate the length of the third (b₁ or b₂) length using Theorem of Pythagoras, i.e. (c₁² -a² = b₁²) and (c₂² - a² = b₂²). b₁ + b₂ + crotch part width can then be used to calculate the distance between E1 and E2. More details on these calculations are described elsewhere herein.

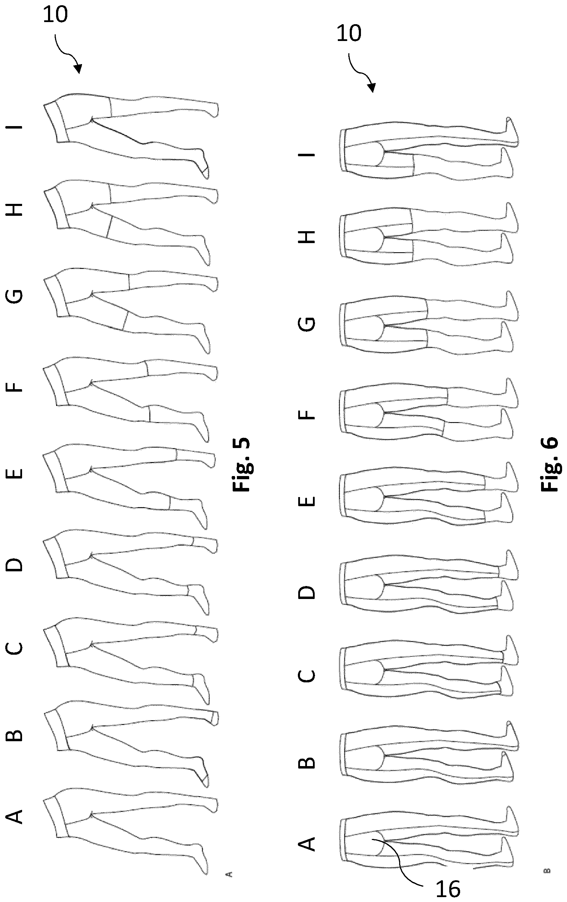

Fig. 5 schematically shows (A to I) oblique frontal views of different embodiments of the compression garment 10 of the invention having different leg lengths and closed-toe (A) and open toe configuration (B to I), wherein each embodiment is adapted to be worn by a female wearer. It can be seen that the compression garment 10 can have the same length in both legs and the same toe configuration as shown in A to H. Alternatively, the compression garment 10 can have one hip-to-leg portion that is longer than the other as shown in part (I). This is particularly advantageous for wearers who have need for compression therapy only or predominantly in one of the legs. Furthermore, the compression garment 10 can have a closed-toe configuration (A) or an open toe configuration (B). It can include a tights-like foot section (A, B, I) or have its distal end at the ankle of the wearer (C) or above the ankle of the wearer (D to I).

Fig. 6 schematically shows (A to I) oblique rear views of different embodiments of the compression garment of the invention having different leg lengths and closed-toe (A) and open toe configuration (B to I), wherein each embodiment is adapted to be worn by a male wearer. It can be seen that the compression garment 10 can have the same length in both legs and the same toe configuration as shown in A to H. Alternatively, the compression garment 10 can have one hip-to-leg portion that is longer than the other as shown in part (I). This is particularly advantageous for wearers who have need for compression therapy only or predominantly in one of the legs. Furthermore, the compression garment 10 can have a closed-toe configuration (A) or an open toe configuration (B). It can include a tights-like foot section (A, B, I) or have its distal end at the ankle of the wearer (C) or above the ankle of the wearer (D to I).

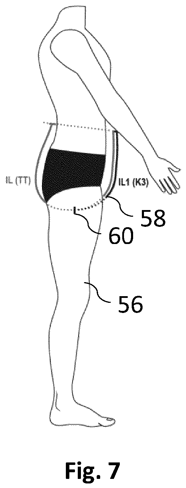

Fig. 7 schematically shows the new total rise length (IL) measurement for compression garments according to the invention. In addition to the usual, well known measurements defined in the RAL norm mentioned elsewhere herein, an additional measurement is performed to measure the distance (IL) between a starting point in the middle of the anterior waistline and an endpoint in the middle of the posterior waistline, wherein the measurement is taken through the crotch area (termed "total rise length"). A regular measuring tape may be used for this measurement, but it has been found in the context of the invention that it is particularly advantageous to use a measuring tape 58 comprising a slider 60. The medical personnel taking the measurements or the wearer himself/herself can fix the slider 60 in the middle of the crotch area and, thereby, measure at the same time the entire distance between posterior and anterior waistline (IL) - i.e. the total rise length - and the distance between the each waistline and the middle of the crotch area. This allows, in particular, simultaneous measurement of the distances IL (total rise length) and IL1 (front rise length). The front rise length IL1 is the length between the starting point in the middle of the anterior waistline and the middle of the crotch area (e.g. as labeled by the slider). The total rise length IL is also known as the measurement "TT". The front rise length IL1 is also known as the measurement "K3".

These measurements can then influence the shape and dimensions of the compression garment, which is particularly beneficial for patients with an unusual ratio between the size of the belly area and the size of the rear (buttocks and hip) area.

Although the present invention has been described in detail with reference to the exemplary embodiments, it is obvious to those skilled in the art that the invention is not restricted to these exemplary embodiments, but rather that modifications can be made in such a way that individual features are omitted or other combinations of the individual features presented are realized, provided that the scope of protection of the appended claims is not exceeded. The present disclosure includes any and all combinations of the individual features presented.

### REFERENCE SIGNS LIST

| | | | |
|---|---|---|---|
| 10 | compression garment | W | maximal width insert |
| 12 | pants section | W' | maximal width of the rear side |
| 14 | rear side | E1 | connection end point |
| 16 | insert | E2 | connection end point |
| 18 | first side section | H | height |
| 20 | second side section | IL | total rise length |
| 22 | first side | IL1 | front rise length |
| 24 | second side | IK | crotch height |
| 26 | buttocks | IH | hip height |
| 26' | first buttock | | |
| 26" | second buttock | | |
| 28 | knitted fabric | | |
| 30 | first seam | | |
| 32 | second seam | | |
| 33 | waistband | | |
| 34 | waistband section | | |
| 36 | intergluteal cleft | | |
| 38 | first hip-to-leg portion | | |
| 40 | second hip-to-leg portion | | |
| 42 | first leg section | | |
| 44 | second leg section | | |
| 46 | crotch part of the insert | | |
| 48 | distal rear section | | |
| 50 | proximal rear section | | |
| 52 | fly opening | | |
| 54 | fly part | | |
| 56 | wearer | | |
| 58 | measuring tape | | |
| 60 | slider | | |

## Claims

1. Compression garment (10) comprising a pants section (12) with a rear side (14), wherein the rear side (14) comprises an insert (16), a first side section (18) and a second side section (20),
the first side section (18) being connected to a first side (22) of the insert (16),
the second side section (20) being connected to a second side (24) of the insert (16),
wherein the insert (16) is adapted to extend over at least part of both buttocks (26) of a wearer,
wherein the insert (16) consists of a seamless knitted fabric (28); and
wherein the insert (16) is located between the first side section (18) and the second side section (20) and a waistband section (34); or the insert (16) is located between the first side section (18) and the second side section (20) and forms part of the upper end of the garment (10).

2. Compression garment (10) according to claim 1, wherein the first side section (18) is connected to the first side (22) of the insert (16) by means of a first seam (30) and the second side section (20) is connected to the second side (24) of the insert (16) by means of a second seam (32) and the first side (22) of the insert (16) is preferably opposite the second side (24) of the insert (16).

3. Compression garment (10) according to any of the preceding claims, the insert (16) having a maximal width (W) that is 30% or more of the maximal width (W) of the rear side (14).

4. Compression garment (10) according to any of the preceding claims, wherein the first side section (18) is adapted to cover a part of a first buttock (26') of the wearer and the second side section (20) is adapted to cover a part of a second buttock (26') of the wearer.

5. Compression garment (10) according to any of the preceding claims, wherein the insert (16) is adapted to extend over at least part of the intergluteal cleft (36) of the wearer.

6. Compression garment (10) according to any of the preceding claims, wherein the rear side (14) comprises the waistband section (34) or wherein the rear side (14) consists of the insert (16), the first side section (18), the second side section (20), the waistband section (34) and seams connecting the aforementioned sections.

7. Compression garment (10) according to any of the preceding claims, wherein the knitted fabric (28) is a flat-knitted fabric.

8. Compression garment (10) according to any of the preceding claims, wherein the garment (10) is a pair of pants or tights.

9. Compression garment (10) according to any of the preceding claims, wherein
the first side section (18) is a part of a first seamless hip-to-leg portion (38),
the second side section (20) is a part of a second seamless hip-to-leg portion (40), and
the first and second hip-to-leg portions (38, 40) are each adapted to extend from the hip region of a wearer to at least the thigh region of the wearer.

10. Compression garment (10) according to any of claims 2 to 9, wherein the first seam (30) and the second seam (32) each extend from the pants section (12) into a first leg section (42) and a second leg section (44), respectively and optionally the first leg section (42) is a part of a first seamless hip-to-leg portion (38) and the second leg section (44) is a part of a second seamless hip-to-leg portion (40), preferably wherein in the first and second leg section (42, 44), respectively,
the first seam (30) connects a first side of the first hip-to-leg portion (38) with a second side of the first hip-to-leg portion (38), and
the second seam (32) connects a first side of the second hip-to-leg portion (40) with a second side of the second hip-to-leg portion (40).

11. Compression garment (10) according to any of the preceding claims, wherein the first side section (18) and the second side section (20) each consist of a seamless knitted fabric (28).

12. Method for determining measurement information for manufacturing a compression garment (10), preferably a compression garment according to any of claims 1 to 11, comprising
a. selecting a wearer in need of compression therapy with compression pants and/or compression tights;
b. acquiring measurement information from the wearer, wherein the measurement information comprises
i. optionally, circumference measurements obtained in the torso, hip, and/or leg area of the wearer;
ii. optionally, length measurements obtained in the torso, hip, and/or leg area of the wearer; and
iii. a measurement of the total rise length (IL) of the wearer and a simultaneous measurement of the front rise length (IL1) of the wearer, wherein the total rise length is the distance between a starting point in the middle of the anterior waistline and an endpoint in the middle of the posterior waistline, wherein the measurement is taken through the crotch area, and the front rise length is the distance between the starting point in the middle of the anterior waistline and the middle of the crotch area; and
c. optionally, manufacturing a compression garment (10) based on the measurement information acquired in step (b) or selecting a pre-fabricated compression garment (10) based on the measurement information acquired in step (b).

13. Method for determining measurement information according to claim 12, wherein:
a. the total rise length (IL) is the distance between a starting point in the middle of the anterior waistline of the wearer and an endpoint in the middle of the posterior waistline of the wearer, wherein the measurement is taken through the crotch area of the wearer; and wherein the front rise length (IL1) is the distance between the starting point in the middle of the anterior waistline of the wearer and the middle of the crotch area of the wearer; and/or
b. the simultaneous measurements in step (b)(iii) use a measuring tape comprising a slider and the slider is placed in the middle of the crotch area; and/or
c. the acquisition of measurement information in step (b)(ii) comprises measuring the length of the crotch height (IK), optionally the acquisition of measurement information in step (b)(ii) comprises measuring a hip height (IH) in the hip area, wherein the hip height (IH) is at least 3 cm higher than the crotch height (IK) and, preferably, taken at the hip height where the hip has the largest circumference.

14. Method for manufacturing a compression garment (10) according to any of claims 1 to 11, comprising
a. obtaining measurement information from a wearer as described in claim or claim 13, or obtaining measurement information for a pre-designed compression garment;
b. fabricating the compression garment (10) based on the measurement information, by connecting three or more pieces of seamless knitted fabric and thereby forming the compression garment,
wherein the three or more pieces of seamless knitted fabric include at least
• a piece of seamless knitted fabric forming an insert (16);
• a piece of seamless knitted fabric forming a first hip-to-leg portion (38);
• a piece of seamless knitted fabric forming a second hip-to-leg portion (40); and
• optionally, a piece of seamless knitted fabric forming a waistband (35); and
wherein the compression garment (10) fabricated in step (b) comprises a pants section (12) with a rear side (14),
wherein the rear side (14) comprises the insert (16), a first side section (18) and a second side section (20), the first side section (18) being connected to a first side (22) of the insert (16), the second side section (20) being connected to a second side (24) of the insert (16), and
wherein the insert (16) is adapted to extend over at least part of both buttocks (26) of a wearer.

15. Method for manufacturing a compression garment (10) according to claim 14, wherein the knitted fabric is a double layer knitted fabric and/or is manufactured in a step (a1) on a knitting machine having at least two needle beds (V, H), wherein the knitted fabric comprises a first layer made of a hydrophilic yarn on the first needle bed (V), a second layer made of a hydrophobic yarn on the second needle bed (H) and the second layer comprises spaced apart split stitches made of the hydrophilic yarn.

## Patentansprüche

1. Kompressionskleidung (10), umfassend einen Hosenteil (12) mit einer Rückseite (14), wobei die Rückseite (14) einen Einsatz (16), einen ersten Seitenabschnitt (18) und einen zweiten Seitenabschnitt (20) umfasst, wobei
der erste Seitenabschnitt (18) mit einer ersten Seite (22) des Einsatzes (16) verbunden ist,
der zweite Seitenabschnitt (20) mit einer zweiten Seite (24) des Einsatzes (16) verbunden ist,
wobei der Einsatz (16) so angepasst ist, dass er sich über mindestens einen Teil beider Gesäßbacken (26) eines Trägers erstreckt,
wobei der Einsatz (16) aus einem nahtlosen Strickstoff (28) besteht; und
wobei der Einsatz (16) zwischen dem ersten Seitenabschnitt (18) und dem zweiten Seitenabschnitt (20) und einem Taillenbundabschnitt (34) angeordnet ist; oder der Einsatz (16) zwischen dem ersten Seitenabschnitt (18) und dem zweiten Seitenabschnitt (20) angeordnet ist und einen Teil des oberen Endes des Kleidungsstücks (10) bildet.

2. Kompressionskleidung (10) nach Anspruch 1, wobei der erste Seitenabschnitt (18) mit der ersten Seite (22) des Einsatzes (16) mittels einer ersten Naht (30) verbunden ist und der zweite Seitenabschnitt (20) mit der zweiten Seite (24) des Einsatzes (16) mittels einer zweiten Naht (32) verbunden ist und die erste Seite (22) des Einsatzes (16) vorzugsweise der zweiten Seite (24) des Einsatzes (16) gegenüberliegt.

3. Kompressionskleidung (10) nach einem der vorstehenden Ansprüche, wobei der Einsatz (16) eine maximale Breite (W) aufweist, die 30 % oder mehr der maximalen Breite (W) der Rückseite (14) beträgt.

4. Kompressionskleidung (10) gemäß einem der vorstehenden Ansprüche, wobei der erste Seitenabschnitt (18) so angepasst ist, dass er einen Teil einer ersten Gesäßbacke (26') des Trägers bedeckt, und der zweite Seitenabschnitt (20) so angepasst ist, dass er einen Teil einer zweiten Gesäßbacke (26') des Trägers bedeckt.

5. Kompressionskleidung (10) nach einem der vorstehenden Ansprüche, wobei der Einsatz (16) so angepasst ist, dass er sich über mindestens einen Teil der Gesäßspalte (36) des Trägers erstreckt.

6. Kompressionskleidung (10) nach einem der vorstehenden Ansprüche, wobei die Rückseite (14) den Taillenbundabschnitt (34) umfasst oder wobei die Rückseite (14) aus dem Einsatz (16), dem ersten Seitenabschnitt (18), dem zweiten Seitenabschnitt (20), dem Taillenbundabschnitt (34) und Nähten besteht, die die vorgenannten Abschnitte verbinden.

7. Kompressionskleidung (10) nach einem der vorstehenden Ansprüche, wobei der Strickstoff (28) ein Flachstrickstoff ist.

8. Kompressionskleidung (10) gemäß einem der vorstehenden Ansprüche, wobei die Kleidung (10) eine Hose oder Strumpfhose ist.

9. Kompressionskleidung (10) gemäß einem der vorstehenden Ansprüche, wobei
der erste Seitenabschnitt (18) ein Teil eines ersten nahtlosen Hüft-zu-Bein-Abschnitts (38) ist,
der zweite Seitenabschnitt (20) ein Teil eines zweiten nahtlosen Hüft-zu-Bein-Abschnitts (40) ist und
die ersten und zweiten Hüft-zu-Bein-Abschnitte (38, 40) jeweils so angepasst sind, dass sie sich von dem Hüftbereich eines Trägers bis mindestens zu dem Oberschenkelbereich des Trägers erstrecken.

10. Kompressionskleidung (10) gemäß einem der Ansprüche 2 bis 9, wobei die erste Naht (30) und die zweite Naht (32) sich jeweils von dem Hosenteil (12) in einen ersten Beinabschnitt (42) bzw. einen zweiten Beinabschnitt (44) erstrecken, und optional der erste Beinabschnitt (42) ein Teil eines ersten nahtlosen Hüft-zu-Bein-Abschnitts (38) ist und der zweite Beinabschnitt (44) ein Teil eines zweiten nahtlosen Hüft-zu-Bein-Abschnitts (40) ist, wobei vorzugsweise in dem ersten bzw. zweiten Beinabschnitt (42, 44)
die erste Naht (30) eine erste Seite des ersten Hüft-Bein-Abschnitts (38) mit einer zweiten Seite des ersten Hüft-Bein-Abschnitts (38) verbindet und
die zweite Naht (32) eine erste Seite des zweiten Hüft-Bein-Abschnitts (40) mit einer zweiten Seite des zweiten Hüft-Bein-Abschnitts (40) verbindet.

11. Kompressionskleidung (10) nach einem der vorstehenden Ansprüche, wobei der erste Seitenabschnitt (18) und der zweite Seitenabschnitt (20) jeweils aus einem nahtlosen Strickstoff (28) bestehen.

12. Verfahren zur Bestimmung von Messinformationen für die Herstellung eines Kompressionskleidungsstücks (10), vorzugsweise eines Kompressionskleidungsstücks gemäß einem der Ansprüche 1 bis 11, umfassend
a. Auswahl eines Trägers, der eine Kompressionstherapie mit Kompressionshosen und/oder Kompressionsstrumpfhosen benötigt;
b. Erfassung von Messinformationen vom Träger, wobei die Messinformationen umfassen
i. optionales Umfangmessen, das im Rumpf-, Hüft- und/oder Beinbereich des Trägers ausgeführt wird;
ii. optionales Längenmessen, das im Rumpf-, Hüft- und/oder Beinbereich des Trägers ausgeführt wird; und
iii. Messen der gesamten Leibhöhe (IL) des Trägers und gleichzeitiges Messen der vorderen Leibhöhe (IL1) des Trägers, wobei die gesamte Leibhöhe der Abstand zwischen einem Anfangspunkt in der Mitte der vorderen Taille und einem Endpunkt in der Mitte der hinteren Taille ist, wobei die Messung durch den Schrittbereich erfolgt, und die Länge der vorderen Leibhöhe der Abstand zwischen dem Anfangspunkt in der Mitte der vorderen Taille und der Mitte des Schrittbereichs ist; und
c. optionales Anfertigen eines Kompressionskleidungsstücks (10) auf der Grundlage der in Schritt (b) erfassten Messinformationen oder Auswahl eines vorgefertigten Kompressionskleidungsstücks (10) auf der Grundlage der in Schritt (b) erfassten Messinformationen.

13. Verfahren zur Bestimmung von Messinformationen gemäß Anspruch 12, wobei:
a. die gesamte Leibhöhe (IL) der Abstand zwischen einem Anfangspunkt in der Mitte der vorderen Taille des Trägers und einem Endpunkt in der Mitte der hinteren Taille des Trägers ist, wobei die Messung durch den Schrittbereich des Trägers erfolgt; und wobei die vordere Leibhöhe (IL1) der Abstand zwischen dem Startpunkt in der Mitte der vorderen Taille des Trägers und der Mitte des Schrittbereichs des Trägers ist; und/oder
b. das gleichzeitige Messen in Schritt (b)(iii) mit einem Maßband erfolgt, das einen Schieber umfasst, und der Schieber in der Mitte des Schrittbereichs platziert wird; und/oder
c. das Erfassen von Messinformationen in Schritt (b)(ii) das Messen der Länge der Schrittlänge (IK) umfasst, optional das Erfassen von Messinformationen in Schritt (b)(ii) das Messen einer Hüfthöhe (IH) im Hüftbereich umfasst, wobei die Hüfthöhe (IH) mindestens 3 cm höher ist als die Schrittlänge (IK) und vorzugsweise an der Hüfthöhe gemessen wird, an der die Hüfte den größten Umfang aufweist.

14. Verfahren zur Herstellung eines Kompressionskleidungsstücks (10) gemäß einem der Ansprüche 1 bis 11, umfassend
a. Erhalten von Messinformationen von einem Träger, wie in Anspruch 12 oder Anspruch 13 beschrieben, oder Erhalten von Messinformationen für ein vorgefertigtes Kompressionsbekleidungsstück;
b. Herstellen des Kompressionsbekleidungsstücks (10) auf der Grundlage der Messinformationen durch Verbinden von drei oder mehr Stücken nahtlosen Strickstoffs und dadurch Formen des Kompressionsbekleidungsstücks, wobei die drei oder mehr Stücke nahtlosen Strickstoffs mindestens umfassen
• ein Stück nahtlosen Strickstoffs, das einen Einsatz (16) bildet;
• ein Stück nahtlosen Strickstoffs, das einen ersten Hüft-zu-Bein-Abschnitt (38) bildet;
• ein Stück nahtlosen Strickstoffs, das einen zweiten Hüft-zu-Bein-Abschnitt (40) bildet; und
• optional ein Stück nahtlosen Strickstoffs, das einen Taillenbund (35) bildet; und
wobei die in Schritt (b) hergestellte Kompressionskleidung (10) einen Hosenabschnitt (12) mit einer Rückseite (14) umfasst,
wobei die Rückseite (14) den Einsatz (16), einen ersten Seitenabschnitt (18) und einen zweiten Seitenabschnitt (20) umfasst, wobei der erste Seitenabschnitt (18) mit einer ersten Seite (22) des Einsatzes (16) verbunden ist, wobei der zweite Seitenabschnitt (20) mit einer zweiten Seite (24) des Einsatzes (16) verbunden ist, und
wobei der Einsatz (16) so angepasst ist, dass er sich über mindestens einen Teil beider Gesäßbacken (26) eines Trägers erstreckt.

15. Verfahren zur Herstellung eines Kompressionskleidungsstücks (10) nach Anspruch 14, wobei der Strickstoff ein zweilagiger Strickstoff ist und/oder in einem Schritt (a1) auf einer Strickmaschine mit mindestens zwei Nadelbetten (V, H) hergestellt wird, wobei der Strickstoff eine erste Lage aus einem hydrophilen Garn auf dem ersten Nadelbett (V), eine zweite Lage aus einem hydrophoben Garn auf dem zweiten Nadelbett (H) umfasst und die zweite Lage beabstandete Spaltmaschen aus dem hydrophilen Garn umfasst.

## Revendications

1. Vêtement de compression (10) comprenant une section de culotte (12) dotée d'un côté arrière (14), dans lequel le côté arrière (14) comprend un insert (16), une première section latérale (18) et une seconde section latérale (20),
la première section latérale (18) étant reliée à un premier côté (22) de l'insert (16),
la seconde section latérale (20) étant reliée à un second côté (24) de l'insert (16),
dans lequel l'insert (16) est adapté pour s'étendre sur au moins une partie des deux fesses (26) d'un porteur, dans lequel l'insert (16) est constitué d'un tissu tricoté sans couture (28) ; et
dans lequel l'insert (16) est situé entre la première section latérale (18) et la seconde section latérale (20) et une section de ceinture (34) ; ou l'insert (16) est situé entre la première section latérale (18) et la seconde section latérale (20) et fait partie de l'extrémité supérieure du vêtement (10).

2. Vêtement de compression (10) selon la revendication 1, dans lequel la première section latérale (18) est reliée au premier côté (22) de l'insert (16) au moyen d'une première couture (30) et la seconde section latérale (20) est reliée au second côté (24) de l'insert (16) au moyen d'une seconde couture (32), et le premier côté (22) de l'insert (16) est de préférence opposé au second côté (24) de l'insert (16).

3. Vêtement de compression (10) selon l'une quelconque des revendications précédentes, l'insert (16) présentant une largeur maximale (W) qui est de 30 % ou plus de la largeur maximale (W) du côté arrière (14).

4. Vêtement de compression (10) selon l'une quelconque des revendications précédentes, dans lequel la première section latérale (18) est adaptée pour couvrir une partie d'une première fesse (26') du porteur, et la seconde section latérale (20) est adaptée pour couvrir une partie d'une seconde fesse (26') du porteur.

5. Vêtement de compression (10) selon l'une quelconque des revendications précédentes, dans lequel l'insert (16) est adapté pour s'étendre sur au moins une partie du sillon interfessier (36) du porteur.

6. Vêtement de compression (10) selon l'une quelconque des revendications précédentes, dans lequel le côté arrière (14) comprend la section de ceinture (34) ou dans lequel le côté arrière (14) consiste en l'insert (16), la première section latérale (18), la seconde section latérale (20), la section de ceinture (34) et des coutures reliant les sections mentionnées ci-dessus.

7. Vêtement de compression (10) selon l'une quelconque des revendications précédentes, dans lequel le tissu tricoté (28) est un tissu tricoté plat.

8. Vêtement de compression (10) selon l'une quelconque des revendications précédentes, dans lequel le vêtement (10) est une paire de culottes ou de collants.

9. Vêtement de compression (10) selon l'une quelconque des revendications précédentes ;
la première section latérale (18) fait partie d'une première partie de hanche à jambe sans couture (38),
la seconde section latérale (20) fait partie d'une seconde partie de hanche à jambe sans couture (40), et
les première et seconde parties de hanche à jambe (38, 40) sont chacune adaptées pour s'étendre de la région de hanche d'un porteur jusqu'au moins la région de cuisse du porteur.

10. Vêtement de compression (10) selon l'une quelconque des revendications 2 à 9, dans lequel la première couture (30) et la seconde couture (32) s'étendent chacune à partir de la section de culotte (12) jusque dans une première section de jambe (42) et une seconde section de jambe (44), respectivement, et facultativement la première section de jambe (42) fait partie d'une première partie de hanche à jambe sans couture (38) et la seconde section de jambe (44) fait partie d'une seconde partie de hanche à jambe sans couture (40), de préférence dans lequel dans la première et la seconde section de jambe (42, 44), respectivement,
la première couture (30) relie un premier côté de la première partie de hanche à jambe (38) à un second côté de la première partie de hanche à jambe (38), et
la seconde couture (32) relie un premier côté de la seconde partie de hanche à jambe (40) à un second côté de la seconde partie de hanche à jambe (40).

11. Vêtement de compression (10) selon l'une quelconque des revendications précédentes, dans lequel la première section latérale (18) et la seconde section latérale (20) consistent chacune en un tissu tricoté sans couture (28).

12. Procédé de détermination d'informations de mesure pour la fabrication d'un vêtement de compression (10), de préférence un vêtement de compression selon l'une quelconque des revendications 1 à 11, comprenant les étapes consistant à
a. sélectionner un porteur ayant besoin d'une thérapie de compression avec une culotte de compression et/ou un collant de compression ;
b. acquérir des informations de mesure auprès du porteur, dans lequel les informations de mesure comprennent
i. facultativement, des mesures de circonférence obtenues dans la zone du torse, de la hanche et/ou de la jambe du porteur ;
ii. facultativement, des mesures de longueur obtenues dans la zone du torse, de la hanche et/ou de la jambe du porteur ; et
iii. une mesure de la longueur de montée totale (IL) du porteur et une mesure simultanée de la longueur de montée avant (IL1) du porteur, dans lequel la longueur de montée totale est la distance entre un point de départ au milieu de la taille antérieure et un point d'extrémité au milieu de la taille postérieure, dans lequel la mesure est prise à travers la zone d'entrejambe, et la longueur de montée avant est la distance entre le point de départ au milieu de la taille antérieure et le milieu de la zone d'entrejambe ; et
c. facultativement, fabriquer un vêtement de compression (10) sur la base des informations de mesure acquises à l'étape (b) ou sélectionner un vêtement de compression préfabriqué (10) sur la base des informations de mesure acquises à l'étape (b).

13. Procédé de détermination d'informations de mesure selon la revendication 12, dans lequel :
a. la longueur de montée totale (IL) est la distance entre un point de départ au milieu de la taille antérieure du porteur et un point d'extrémité au milieu de la taille postérieure du porteur, dans lequel la mesure est prise à travers la zone d'entrejambe du porteur ; et dans lequel la longueur de montée avant (IL1) est la distance entre le point de départ au milieu de la taille antérieure du porteur et le milieu de la zone d'entrejambe du porteur ; et/ou
b. les mesures simultanées à l'étape (b)(iii) utilisent un ruban à mesurer comprenant un curseur, et le curseur est placé au milieu de la zone d'entrejambe ; et/ou
c. l'acquisition d'informations de mesure à l'étape (b)(ii) comprend une mesure de la longueur de la hauteur d'entrejambe (IK), facultativement l'acquisition d'informations de mesure à l'étape (b)(ii) comprend une mesure d'une hauteur de hanche (IH) dans la zone de hanche, dans lequel la hauteur de hanche (IH) est au moins 3 cm plus élevée que la hauteur d'entrejambe (IK) et, de préférence, prise au niveau de la hauteur de hanche où la hanche présente la plus grande circonférence.

14. Procédé de fabrication d'un vêtement de compression (10) selon l'une quelconque des revendications 1 à 11, comprenant les étapes consistant à
a. obtenir des informations de mesure d'un porteur tel que décrit dans la revendication ou la revendication 13, ou obtenir des informations de mesure pour un vêtement de compression préconçu ;
b. fabriquer le vêtement de compression (10) sur la base des informations de mesure, en reliant trois pièces de tissu tricoté sans couture ou plus, et former ainsi le vêtement de compression,
dans lequel les trois pièces de tissu tricoté sans couture ou plus incluent au moins
• une pièce de tissu tricoté sans couture formant un insert (16) ;
• une pièce de tissu tricoté sans couture formant une première partie de hanche à jambe (38) ;
• une pièce de tissu tricoté sans couture formant une seconde partie de hanche à jambe (40) ; et
• facultativement, une pièce de tissu tricoté sans couture formant une ceinture (35) ; et
dans lequel le vêtement de compression (10) fabriqué à l'étape (b) comprend une section de culotte (12) avec un côté arrière (14),
dans lequel le côté arrière (14) comprend l'insert (16), une première section latérale (18) et une seconde section latérale (20), la première section latérale (18) étant reliée à un premier côté (22) de l'insert (16), la seconde section latérale (20) étant reliée à un second côté (24) de l'insert (16), et
dans lequel l'insert (16) est adapté pour s'étendre sur au moins une partie des deux fesses (26) d'un porteur.

15. Procédé de fabrication d'un vêtement de compression (10) selon la revendication 14, dans lequel le tissu tricoté est un tissu tricoté à deux couches et/ou est fabriqué dans une étape (a1) sur une machine à tricoter présentant au moins deux lits d'aiguilles (V, H), dans lequel le tissu tricoté comprend une première couche constituée d'un fil hydrophile sur le premier lit d'aiguilles (V), une seconde couche constituée d'un fil hydrophobe sur le second lit d'aiguilles (H), et la seconde couche comprend des points fendus espacés constitués du fil hydrophile.
